# EUROPÄISCHE PATENTSCHRIFT

(11) **EP 0 441 193 B1**
(45) Veröffentlichungstag und Bekanntmachung des Hinweises auf die Patenterteilung: **12.04.1995**
(21) Anmeldenummer: 91100936.3
(22) Anmeldetag: 25.01.1991
(51) Int. Cl.: A61B 5/00

(54) **Messgerät zur Bestimmung der Schwitzneigung von Menschen**
Measuring instrument for determining the tendency towards sweating of people
Dispositif de mesure pour déterminer la tendence pour transpirer de l'homme

(30) Priorität: 01.02.1990 DE 9001131 U
(43) Veröffentlichungstag der Anmeldung: 14.08.1991
(73) Patentinhaber: FEMIRA-WERKE GUSSMANN GmbH + Co. KG, MATRATZENFABRIK - MÖBELFABRIK, D-74385 Pleidelsheim (DE)
(72) Erfinder: Vogt, Norbert c/o Universität Kiel, W-2300 Kiel (DE)
(74) Vertreter: Jackisch-Kohl, Anna-Katharina

(56) Entgegenhaltungen:
- DE-A- 1 466 811
- DE-A- 2 912 349
- US-A- 4 860 753

## Beschreibung

Die Erfindung betrifft ein Meßgerät zur Bestimmung der Schwitzneigung von Menschen nach dem Oberbegriff des Anspruches 1.

Zur Aufrechterhaltung der Lebensvorgänge laufen im menschlichen Körper vielfältige Stoffwechselvorgänge ab. Die zugeführte Nahrung wird ab- und umgebaut, während nicht verwertbare Stoffe vom Körper ausgeschieden werden. Bei diesen chemischen Reaktionen entsteht zu einem großen Teil Wärme, die vom Körper zur Aufrechterhaltung einer stabilen Kerntemperatur genutzt wird. Durch Umwelt und Lebensführung bedingt, fällt jedoch unterschiedlich viel Wärme an. Größere Abweichungen von der Soll-Temperatur würden zu einer Schädigung oder gar zum Absterben des Organismus führen.

Durch regulative Mechanismen ist der menschliche Körper in der Lage, diese Schwankungen auszugleichen und für eine relativ konstante Körpertemperatur zu sorgen. Hierbei bedient er sich des Blutgefäßsystems und der Schweißdrüsen. Der Wärmeaustausch erfolgt überwiegend über die Haut. Diese kann durch Wärmestrahlung, Wärmeleitung, Konvexion oder Verdunstung von Schweißsekret erfolgen. Im Körperinneren erfolgt der Transport konvektiv über das Blutgefäßsystem und konduktiv zwischen den Alveolen und der Hautoberfläche.

Der Bereich des subjektiven Wohlbefindens, die sogenannte thermisch neutrale Zone, ist eng begrenzt und beträgt nur wenige 1/10 Grad. Die Hautoberfläche weist eine hierfür charakteristische Temperaturverteilung auf. Die Abgabe von Schweiß bewirkt bei Verdunstung eine effiziente Unterstützung des Wärmeregulationssystems. Kann die an die Hautoberfläche abgeführte Schweißmenge jedoch nicht verdunsten, so beeinträchtigt der dann entstehende Schweißfilm das Behaglichkeitsempfinden.

Eine zu große Wärmeproduktion oder eine zu geringe Wärmeabgabe bewirken eine Erhöhung der Hauttemperatur und damit eng verbunden eine Steigerung der Feuchteabgabe des Körpers.

Der Erfindung liegt die Aufgabe zugrunde, ein Meßgerät zu entwickeln, mit dem sehr einfach und genau die subjektive Reaktion des Menschen auf Wärmereize, d.h. seine Schwitzneigung, ermittelt werden kann.

Diese Aufgabe wird beim gattungsgemäßen Meßgerät erfindungsgemäß mit den kennzeichnenden Merkmalen des Anspruches 1 gelöst.

Zum Meßvorgang greift der Mensch mit seiner Hand durch die Durchgriffsöffnung und erfaßt den Meßkörper im Gehäuse. Die im Meßkörper vorgesehenen Meßelemente kommen mit der Hautoberfläche in Berührung und messen dabei den Hautwiderstand. Er ist von dem in der Handfläche befindlichen Schweiß abhängig. Die Wärmebelastung des Körpers spiegelt sich in der Hautfeuchtigkeit wider, so daß sie als Maß für den thermischen Zustand des Menschen herangezogen werden kann. Die Handfläche ist in besonderer Weise geeignet, die subjektive Reaktion des Menschen auf Wärmereize widerzuspiegeln. In diesem Hautbereich kommen alle eingangs erwähnten Einflußfaktoren gleichzeitig zum Tragen. Ferner wirken sich bei der Hand als exponierter Extremität Temperaturschwankungen des Kern-Schale-Systems am schnellsten und stärksten aus. Die vergleichsweise hohe Schweißdrüsendichte ermöglicht eine hohe Schweißabgabe und damit eine besonders zuverlässige Bestimmung der Schwitzneigung. Mit der Auswerteelektronik werden die von den Meßelementen ermittelten Meßwerte ausgewertet und beispielsweise angezeigt.

DE-A-1.466.811, Figur 3 Zeigt eine Meßsonde zur Bestimmung der Feuchtigkeitsabgabe der Haut, wobei nur die Meßsonde temperaturkompensiert ist.

Weitere Merkmale der Erfindung ergeben sich aus den weiteren Ansprüchen, der Beschreibung und den Zeichnungen.

Die Erfindung wird anhand eines in den Zeichnungen dargestellten Ausführungsbeispieles näher erläutert. Es zeigen
- Fig. 1: eine perspektivische Ansicht des erfindungsgemäßen Meßgerätes,
- Fig. 2: eine Seitenansicht des erfindungsgemäßen Meßgerätes ohne Gehäuseoberteil,
- Fig. 3: in vergrößerter Darstellung einen Meßgriff des erfindungsgemäßen Meßgerätes,
- Fig. 4: ein Schaltbild der Auswerteelektronik des erfindungsgemäßen Meßgerätes.

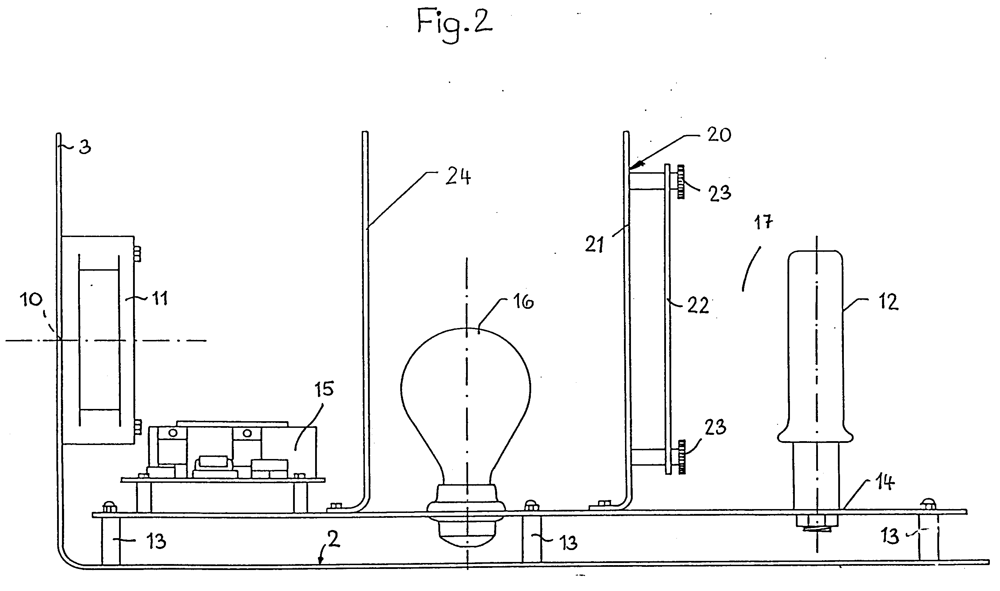

Das Meßgerät hat ein Gehäuse 1 mit einem L-förmigen Bodenteil 2 (Fig. 2), das auch die Rückwand 3 des Gehäuses 1 bildet. Das Bodenteil 2 ist auf der Unter- und Rückseite mit einem Oberteil 4 des Gehäuses verschraubt. Stirnseitig ist an den Gehäuseteilen 2 und 4 eine Frontplatte 5 befestigt, die im Ausführungsbeispiel quadratischen Umriß hat, aber auch jede andere geeignete Umrißform haben kann. Vorzugsweise bestehen die Gehäuseteile 2 und 4 aus Stahlblech und die Frontplatte 5 aus Kunststoff, vorzugsweise aus Acrylglas. Die Frontplatte 5 ist lösbar an den Gehäuseteilen 2 und 4 befestigt, vorzugsweise mit vier an den Ecken vorgesehenen Schrauben 6. Mittig ist die Frontplatte 5 mit einer vorzugsweise kreisförmigen Durchgriffsöffnung 7 versehen. An der Frontplatte 5 befindet sich ein Netzschalter 8, der vorzugsweise eine integrierte Bereitschaftsanzeige hat. Außerdem ist an der Frontplatte 5 eine Anzeige 9 vorgesehen, die vorzugsweise ein Leuchtdioden-Display ist. Um eine gute Ablesbarkeit zu gewährleisten, ist die Anzeige 9 vorteilhaft nahe der Oberseite des Gehäuses 1, vorzugsweise mittig oberhalb der Durchgriffsöffnung 7 angeordnet. Im dargestellten bevorzugten Ausführungsbeispiel ist die Anzeige 9 ein zehnstelliges Display, das sich aus sieben grünen und drei roten Leuchtdioden zusammensetzt. Ein Anzeigewert im roten Bereich der Anzeige 9 signalisiert eine überdurchschnittliche Transpirationsneigung.

An der Rückseite des Gehäuses 1 befindet sich ein (nicht dargestellter) Netzanschluß und eine Öffnung 10 (Fig. 2), hinter der ein Lüfter 11, vorzugsweise ein Radiallüfter, montiert ist. Der Lüfter 11 erzeugt einen kontinuierlichen Luftstrom, der durch das Gehäuse 1 strömt und aus der Durchgriffsöffnung 7 austritt. Der Luftstrom dient einerseits der schnellen Restitution eines im Gehäuse 1 angeordneten Meßgriffes 12 nach der Benutzung, andererseits aber zum Wärmetransport und damit zur Stimulierung der Transpiration des Probanden.

Auf dem Bodenteil 2 ist mittels Distanzstücke 13 eine Grundplatte 14 befestigt, auf der nahe der Rückwand 3 eine Auswerteelektronik 15 befestigt ist. Im Mittelbereich der Grundplatte 14 sind zwei Fassungen für Glühlampen 16 montiert, die der Aufheizung des vom Lüfter 11 erzeugten Luftstromes dienen, so daß sich in der eigentlichen Meßkammer 17, in der sich der Meßgriff 12 befindet, eine erhöhte Temperatur einstellt, die im Ausführungsbeispiel etwa 34°C beträgt. Dieser Wert gilt bei einer Umgebungstemperatur des Meßgerätes von etwa 20°C und einer Austrittsgeschwindigkeit der Luft aus der Durchgriffsöffnung 7 von etwa 0,3 m pro Sekunde.

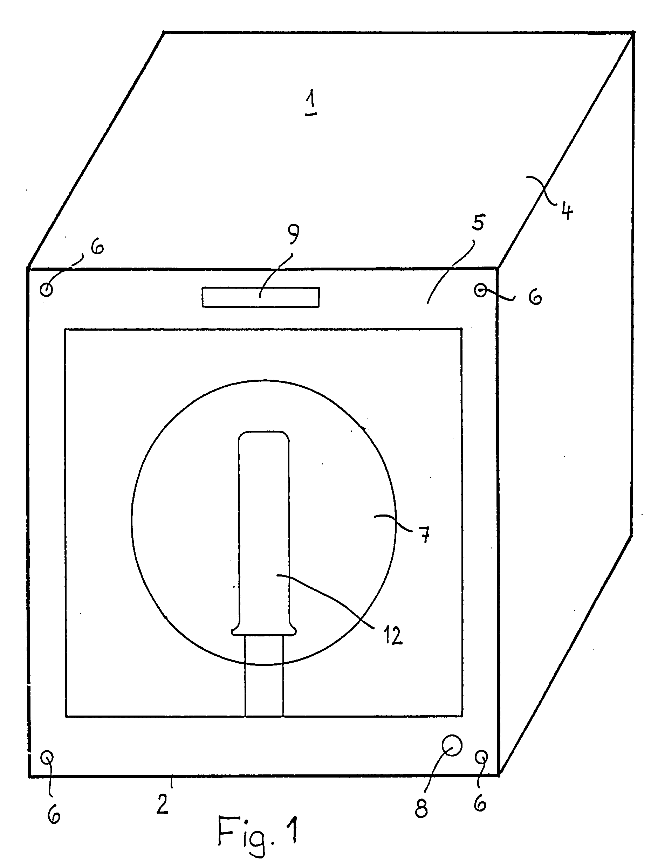

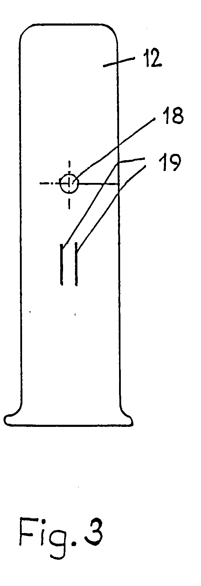

Der Meßgriff 12 ist mittig in bezug auf die Durchgriffsöffnung 7 auf der Grundplatte 14 befestigt (Fig. 1) und trägt Meßelemente 18 und 19 zur Messung des Hautwiderstandes und der Hauttemperatur (Fig. 3). Die Meßelemente 18, 19 sind Sensoren, die so angeordnet sind, daß bei flächigem Umfassen des Meßgriffes 12 mit der Hand ein guter Kontakt zwischen Handfläche und den Meßwertaufnehmern 18, 19 gewährleistet ist. Die Hauttemperaturmessung übernimmt vorzugsweise ein gegen Berührung elektrisch isolierter Thermistor 18, während die Hautwiderstandsmessung vorzugsweise über zwei parallel angeordnete Platindrähte bzw. - elektroden als Meßelemente 19 erfolgt, die - materialbedingt - eine Veränderung des Übergangswiderstandes durch Korrosion ausschließen.

Bezogen auf die Durchgriffsöffnung 7 hinter dem Meßgriff 12 befindet sich ein Blendschutz 20, der eine von den Glühlampen 16 ausgehende Blendwirkung verhindert, andererseits aber den vom Lüfter 11 erzeugten Luftstrom passieren läßt. Der Blendschutz 20 wird durch zwei mit Abstand hintereinander angeordnete Abschirmbleche 21 und 22 gebildet, die ein labyrinthartiges Trennelement bilden. Das den Glühlampen 16 zugewandte Abschirmblech 21 hat mindestens eine für den vom Lüfter 11 erzeugten Luftstrom durchlässige (nicht dargestellte) Öffnung. Das mit Abstand davor angeordnete Abschirmblech 22 ist geschlossen ausgebildet und so groß, daß es, in Achsrichtung der Durchgriffsöffnung 7 gesehen, die Glühlampen 16 verdeckt. Das Abschirmblech 22 hat kleinere Außenabmessungen als das Abschirmblech 21. Dadurch werden zwischen dem Abschirmblech 22 und den Innenseiten des Gehäuses 1 Durchtritte für den Luftstrom gebildet, so daß er in ausreichendem Maße zum Meßgriff 12 strömen kann. Der Luftstrom gelangt zunächst durch die Öffnung des Abschirmbleches 21 auf das Abschirmblech 22, an dem er rechtwinklig abgelenkt wird. Der Luftstrom kann dann zwischen dem Abschirmblech 22 und den Gehäuseinnenseiten weiter nach vorn zum Meßgriff 12 strömen. Das Abschirmblech 22 ist vorzugsweise lösbar, insbesondere mit vier Rändelmuttern 23, am Abschirmblech 21 befestigt, so daß das Abschirmblech 22 zum Auswechseln der Glühlampen 16 bei montiertem Gehäuse 1 abgenommen werden kann. Zwischen den Glühlampen 16 und der Auswerteelektronik 15 befindet sich ein Berührungsschutz 24, der vorzugsweise durch ein Gitter gebildet ist. Der Berührungsschutz 24 verhindert das versehentliche Berühren von spannungsführenden Teilen.

Als Wärmequelle können anstelle der Glühlampen 16 auch andere geeignete Einrichtungen, wie beispielsweise Heizelemente, eingesetzt werden, mit denen die vom Lüfter 11 erzeugte Luft auf den gewünschten Wert aufgeheizt werden kann.

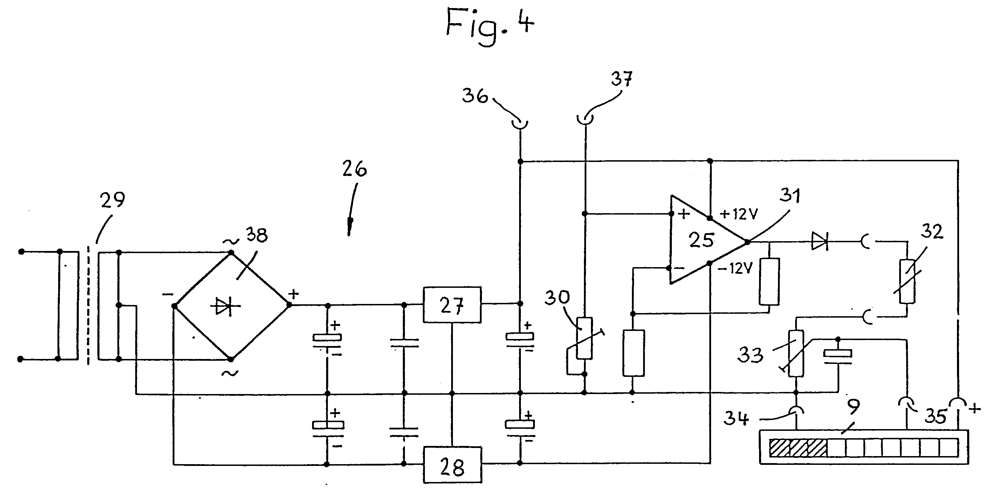

Die zur Anzeige notwendige Aufbereitung des von den Meßelementen 19 erzeugten Meßsignals des Hautwiderstandes erfolgt mittels eines Operationsverstärkers 25 (Fig. 4). Durch eine starke Gegenkopplung arbeitet er als Spannungsfolger, dessen Eingangswiderstand so hochohmig ist, daß er die entsprechenden Hautwiderstandssignale verarbeiten kann.

Eine duale Spannungsversorgung erfolgt über ein Netzteil 26 mit zwei Festspannungsreglern 27 und 28. Das Netzteil 26 hat einen Transformator 29, dem eine Brückenschaltung 38 nachgeschaltet ist und der den VDI-Vorschriften 0551 und der Schutzklasse II entsprechen sollte. Darüber hinaus ist eine Schutzerdung des Gehäuses 1 vorgesehen.

Solange keine leitende Verbindung zwischen den beiden auf dem Meßgriff 12 befindlichen Meßelektroden 19 besteht, also der Meßgriff nicht von einer Hand umgriffen ist, liegt der nichtinvertierende Eingang des Operationsverstärkers 25 über einen regelbaren Widerstand 30 gegen Masse, so daß am Ausgang 31 des Verstärkers 25 kein Ausgangssignal auftritt. Werden die beiden Meßelektroden 19 jedoch über die Haut der Handfläche leitend verbunden, so verschiebt sich das Potential am Eingang des Operationsverstärkers 25 so, daß er ein dem Hautwiderstand proportionales Ausgangssignal liefert. Dieses wird nun entsprechend der an einem regelbaren Widerstand 32 anliegenden, der Hauttemperatur entsprechenden Meßgröße und der Einstellung eines weiteren regelbaren Widerstandes 33 verkleinert und der Anzeige 9 zugeführt.

Durch den erwärmten Luftstrom wird bei der Messung die den Meßgriff 12 umgreifende Hand erwärmt und damit zur Schweißbildung angeregt. Da bevorzugt sowohl der Hautwiderstand als auch die Hauttemperatur gleichzeitig gemessen werden, läßt sich durch Kombination dieser Meßgrößen die Schwitzneigung des Probanden optimal ermitteln. Mit zunehmender Reizstärke ist hierbei eine bessere Differenzierung der Probanden hinsichtlich der Schwitzbereitschaft möglich. Als Meßwerte des Hautwiderstandes ergeben sich bei dem beschriebenen und dargestellten Ausführungsbeispiel Widerstandswerte zwischen 150 k Ω und 9,6 M Ω .

Vor Beginn der Messung muß die Auswerteelektronik 15 abgeglichen werden. Hierzu wird an Meßpunkte 34 und 35 ein Spannungsmesser mit hochohmigem Eingang angeschlossen. Der Widerstand 32 wird mit der erhöhten Temperatur, im Ausführungsbeispiel von 34°C, beaufschlagt, wobei sich ein bestimmter Widerstandswert einstellt. Anschließend wird der Widerstand 30, der ein Trimmer ist, etwa in halbe Schleiferstellung gebracht. Dann werden die Elektrodenanschlüsse 36 und 37 für die Meßelektroden 19 miteinander kurzgeschlossen. Nun wird mit dem Widerstand 33 der Spannungswert zwischen Masse (Meßpunkt 34) und Signaleingang der Anzeige 9 (Meßpunkt 35) auf einen solchen Wert eingestellt, daß alle zehn Leuchdioden der Anzeige 9 aufleuchten. Die Kurzschlußbrücke zwischen den Elektrodenanschlüssen 36 und 37 wird entfernt und durch einen Widerstand mit fester Widerstandsgröße ersetzt. Die Temperatur am Meßelement 18 (Thermistor) muß unverändert bei der erhöhten Temperatur liegen, also im Ausführungsbeispiel 34°C betragen. Mittels des Widerstandes 30 wird nun ein geeigneter Spannungswert zwischen dem Meßpunkt 35 und der Masse 34 eingestellt.

Das auf diese Weise abgeglichene Meßgerät ermöglicht eine orientierende Bestimmung der Schwitzneigung und eine Einordnung in die gesamte Reaktionsbreite. Mit dem Meßgerät läßt sich auf die beschriebene Weise einfach und dennoch sehr genau die Schwitzneigung der Personen bestimmen, wobei bevorzugt in Kombination der Hautwiderstand und die Hauttemperatur an der Handfläche herangezogen werden. Beide Meßgrößen ermöglichen eine exakte Einordnung hinsichtlich der Schwitzbereitschaft der Probanden.

Beim beschriebenen und dargestellten Meßgerät ist ein Meßgriff als Meßkörper 12 vorgesehen. Bei einer anderen, nicht dargestellten Ausführungsform kann der Meßkörper 12 auch als eine Art Schale ausgebildet sein, auf welche die zu messende Hand gelegt wird. Der Meßkörper 12 kann aber auch mit einer kugelförmigen Auflagefläche für die Hand versehen sein. Auf jeden Fall sind diese Meßkörper, abgesehen von der unterschiedlichen Formgebung, gleich ausgebildet wie das beschriebene und dargestellte Ausführungsbeispiel, bei dem der Meßkörper 12 als Griff ausgebildet ist.

Der als Griff ausgebildete Meßkörper 12 muß nicht, wie im Ausführungsbeispiel dargestellt, aufrecht im Gehäuse 1 angeordnet sein, sondern kann selbstverständlich auch schräg oder horizontal im Gehäuse angeordnet sein.

Um die Meßwerte anzuzeigen, kann der jeweilige Meßwert auch durch Zahlen und/oder durch Farbanzeigen angezeigt werden.

Es ist auch möglich, am Gehäuse 1 selbst keine Anzeige 9 mehr vorzusehen, sondern die Auswertung der Messung über einen Monitor im Rahmen einer Computerberatung vorzunehmen.

## Patentansprüche

1. Meßgerät zur Bestimmung der Schwitzneigung von Menschen,
dadurch gekennzeichnet, daß das Meßgerät mit einem Gehäuse (1) versehen ist, das für die Hand mindestens eine Durchgriffsöffnung (7) aufweist, hinter der mindestens ein Meßkörper (12) angeordnet ist, der mindestens ein Hauttemperatur-Meßelement (18) und an seiner Oberfläche Meßelemente (19) zur Messung des Hautwiderstandes aufweist, die an eine Auswerteelektronik (15) angeschlossen sind.

2. Meßgerät nach Anspruch 1,
dadurch gekennzeichnet, daß das Hauttemperatur-Meßelement (18) ein Thermistor ist.

3. Meßgerät nach Anspruch 1 oder 2,
dadurch gekennzeichnet, daß der Meßkörper (12) im Strömungsbereich mindestens eines Lüfters (11) liegt, der vorzugsweise, von der Durchgriffsöffnung (7) aus gesehen, hinter dem Meßkörper (12) angeordnet und an der Rückwand (3) des Gehäuses (1) befestigt ist.

4. Meßgerät nach Anspruch 3,
dadurch gekennzeichnet, daß zwischen dem Lüfter (11) und dem Meßkörper (12) mindestens ein Berührungsschutz (24), vorzugsweise ein Schutzgitter, angeordnet ist, hinter dem vorzugsweise die Auswerteelektronik (25) liegt.

5. Meßgerät nach einem der Ansprüche 1 bis 4,
dadurch gekennzeichnet, daß hinter dem Meßkörper (12), von der Durchgriffsöffnung (7) aus gesehen, mindestens eine Wärmequelle (16) vorgesehen ist, die vorzugsweise mindestens eine Glühlampe ist, die im Luftstrom des Lüfters (11) liegt.

6. Meßgerät nach Anspruch 5,
dadurch gekennzeichnet, daß die Wärmequelle (16) im Bereich zwischen dem Lüfter (11) und dem Meßkörper (12) angeordnet ist, und daß vorzugsweise zwischen der Wärmequelle (16) und dem Lüfter (11) mindestens ein für den Luftstrom durchlässiger Blendschutz (20) liegt.

7. Meßgerät nach Anspruch 6,
dadurch gekennzeichnet, daß der Blendschutz (20) durch zwei mit Abstand hintereinander angeordnete Abschirmbleche (21, 22) gebildet ist, von denen das der Wärmequelle (16) zugewandte Abschirmblech (21) mindestens eine Durchtrittsöffnung für die Wärmestrahlung und den Luftstrom hat, während das andere Abschirmblech (22) geschlossen ausgebildet ist und kleinere Außenabmessungen hat als das hintere Abschirmblech (21).

8. Meßgerät nach einem der Ansprüche 1 bis 7,
dadurch gekennzeichnet, daß an die Auswerteelektronik (15) mindestens eine Anzeige (9), vorzugsweise ein Display, angeschlossen ist.

9. Meßgerät nach einem der Ansprüche 1 bis 8,
dadurch gekennzeichnet, daß das Meßelement (19) zur Messung des Hautwiderstandes eine Elektrode, vorzugsweise eine Platinelektrode, ist.

10. Meßgerät nach einem der Ansprüche 1 bis 9,
dadurch gekennzeichnet, daß der Meßkörper (12) als Griff ausgebildet ist.

11. Meßgerät nach einem der Ansprüche 1 bis 9,
dadurch gekennzeichnet, daß der Meßkörper (12) schalenartig ausgebildet ist.

12. Meßgerät nach einem der Ansprüche 1 bis 9,
dadurch gekennzeichnet, daß der Meßkörper (12) eine kugelförmige Auflagefläche für die Hand aufweist.

## Claims

1. A measuring device for determining a person's tendency to sweat, **characterised in that** the measuring device is provided with a casing (1) having at least one opening (7) for a hand to reach through, at least one measuring body (12) being arranged behind the said opening and having at least one skin-temperature-measuring member (18), and measuring members (19) on its surface, to measure the resistance of the skin, the said members being connected to evaluating electronics (15).

2. A measuring device in accordance with claim 1, **characterised in that** the skin-temperature-measuring member (18) is a thermistor.

3. A measuring device in accordance with claim 1 or 2, **characterised in that** the measuring body (12) is positioned within the range of flow from at least one ventilator (11) which is preferably arranged behind the measuring body (12), as seen from the opening (7), and secured to the rear wall (3) of the casing (1).

4. A measuring device in accordance with claim 3, **characterised in that** at least one member (24) to protect against accidental contact, for example a protective mesh, is arranged between the ventilator (11) and the measuring body (12), and the evaluating electronics (25) [*sic*] are preferably arranged behind the protective member.

5. A measuring device in accordance with one of claims 1 to 4, **characterised in that** at least one heat source (16) is provided behind the measuring body (12), as seen from the opening (7), and the said source is preferably at least one incandescent bulb positioned in the flow of air from the ventilator (11).

6. A measuring device in accordance with claim 5, **characterised in that** the heat source (16) is arranged in the region between the ventilator (11) and the measuring body (12), and at least one anti-glare device (20), which is permeable to the flow of air, is preferably positioned between the heat source (16) and the ventilator (11).

7. A measuring device in accordance with claim 6, **characterised in that** the anti-glare device (20) is formed from two sheet-metal shields (21, 22) arranged one behind the other with a separating distance therebetween, and the sheet-metal shield (21) facing the heat source (16) has at least one through opening for heat radiation and the flow of air, whilst the other sheet-metal shield (22) is formed to be continuous and has smaller outer dimensions than the rear sheet-metal shield (21).

8. A measuring device in accordance with one of claims 1 to 7, **characterised in that** at least one indicator (9), preferably a display, is connected to the evaluation electronics (15).

9. A measuring device in accordance with one of claims 1 to 8, **characterised in that** the measuring member (19) for measuring the resistance of the skin is an electrode, preferably a platinum electrode.

10. A measuring device in accordance with one of claims 1 to 9, **characterised in that** the measuring body (12) is formed as a handle.

11. A measuring device in accordance with one of claims 1 to 9, **characterised in that** the measuring body (12) is formed to be shell-like.

12. A measuring device in accordance with one of claims 1 to 9, **characterised in that** the measuring body (12) has a spherical surface to support the hand.

## Revendications

1. Appareil de mesure pour la détermination de la tendance à la transpiration d'individus, caractérisé en ce que l'appareil de mesure est pourvu d'un boîtier (1), qui présente au moins une ouverture de passage (7) pour la main, derrière laquelle est agencé au moins un corps de mesure (12), qui présente au moins un élément de mesure de la température de la peau (18) et, sur sa surface, des éléments de mesure (19) pour la mesure de la résistance de la peau, qui sont raccordés à un système électronique d'évaluation (15).

2. Appareil de mesure selon la revendication 1, caractérisé en ce que l'élément de mesure de la température de la peau (18) est une thermistance.

3. Appareil de mesure selon la revendication 1 ou 2, caractérisé en ce que le corps de mesure (12) se situe dans la zone d'écoulement d'au moins un ventilateur (11) qui, vu de l'ouverture de passage (7), est de préférence agencé derrière le corps de mesure (12) et qui est fixé sur la paroi arrière (3) du boîtier (1).

4. Appareil de mesure selon la revendication 3, caractérisé en ce qu'au moins une protection anti-contact (24), de préférence une grille protectrice, est agencée entre le ventilateur (11) et le corps de mesure (12), derrière laquelle se situe de préférence le système électronique d'évaluation (15).

5. Appareil de mesure selon l'une des revendications 1 à 4, caractérisé en ce qu'au moins une source de chaleur (16) est prévue derrière le corps de mesure (12), vue de l'ouverture de passage (7), ladite source de chaleur (16) étant de préférence au moins une lampe à incandescence, qui se situe dans le courant d'air du ventilateur (11).

6. Appareil de mesure selon la revendication 5, caractérisé en ce que la source de chaleur (16) est agencée dans la région entre le ventilateur (11) et le corps de mesure (12), et en ce qu'au moins un antiéblouissant (20) perméable au courant d'air est situé, de préférence, entre la source de chaleur (16) et le ventilateur (11).

7. Appareil de mesure selon la revendication 6, caractérisé en ce que l'antiéblouissant (20) est formé de deux tôles de protection (21, 22) agencées à distance l'une derrière l'autre, parmi lesquelles la tôle de protection (21) tournée vers la source de chaleur (16) a au moins une ouverture de passage pour le rayonnement de chaleur et le courant d'air, tandis que l'autre tôle de protection (22) est réalisée fermée et a de plus petites dimensions extérieures que la tôle de protection (21) postérieure.

8. Appareil de mesure selon l'une des revendications 1 à 7, caractérisé en ce qu'au moins un affichage (9) est raccordé au système électronique d'évaluation (15), de préférence un affichage électronique.

9. Appareil de mesure selon l'une des revendications 1 à 8, caractérisé en ce que l'élément de mesure (19) pour la mesure de la résistance de la peau est une électrode, de préférence une électrode en platine.

10. Appareil de mesure selon l'une des revendications 1 à 9, caractérisé en ce que le corps de mesure (12) est réalisé sous la forme d'une poignée.

11. Appareil de mesure selon l'une des revendications 1 à 9, caractérisé en ce que le corps de mesure (12) est réalisé sous la forme d'une coquille.

12. Appareil de mesure selon l'une des revendications 1 à 9, caractérisé en ce que le corps de mesure (12) présente une surface d'appui pour la main en forme de sphère.
